# EUROPEAN PATENT SPECIFICATION

(11) **EP 3 865 570 B1**
(45) Date of publication and mention of the grant of the patent: **25.06.2025**
(21) Application number: 19867367.5
(22) Date of filing: 19.09.2019
(51) Int. Cl.: C12N 5/071, A61K 35/36, A61L 27/36, A61L 27/38, A61L 27/60, A61P 17/14

(54) **IN VITRO GROWTH METHOD FOR HAIR FOLLICULAR EPITHELIAL STEM CELLS**
IN-VITRO-WACHSTUMSVERFAHREN FÜR HAARFOLLIKEL-EPITHELSTAMMZELLEN
PROCÉDÉ DE CROISSANCE IN VITRO DE CELLULES SOUCHES ÉPITHÉLIALES DE FOLLICULES PILEUX

(30) Priority: 26.09.2018 JP 2018179816
(43) Date of publication of application: 18.08.2021
(73) Proprietor: Organ Technologies, Inc., Minato-ku Tokyo 105-0001 (JP); RIKEN, Wako-shi, Saitama 351-0198 (JP)
(72) Inventor: TSUJI, Takashi, Wako-shi, Saitama 351-0198 (JP); ASAKAWA, Kyosuke, Wako-shi, Saitama 351-0198 (JP); TAKEO, Makoto, Wako-shi, Saitama 351-0198 (JP); TOYOSHIMA, Koh-ei, Tokyo 105-0001 (JP); OGAWA, Miho, Tokyo 105-0001 (JP); MORI, Yutaka, Kyoto-shi, Kyoto 612-8501 (JP); KISHIMOTO, Kyosuke, Kyoto-shi, Kyoto 612-8501 (JP)
(74) Representative: V.O.
(86) International application number: PCT/JP2019/036687
(87) International publication number: WO 2020/066818

(56) References cited:
- WO-A1-2012/108069
- WO-A1-2012/115079
- WO-A1-2017/060240
- JP-A- 2007 274 949
- JP-A- 2012 249 556
- JP-A- 2017 524 684
- JP-A- 2017 536 411
- JP-A- 2019 135 947
- JP-A- H 111 416
- US-A1- 2009 198 336

## Description

### TECHNICAL FIELD

The present invention relates to a culture medium for efficient growth of epithelial cells capable of being used to manufacture regenerated hair follicle germs and to a method for growth of such epithelial cells using the same.

### BACKGROUND ART

To make hair regenerative medicine a reality, acquisition of mesenchymal stem cells and epithelial stem cells possessing hair follicle inductivity required for reconstruction of hair follicle germs, as well as the *in vitro* growth thereof, are necessary. With regard to mesenchymal stem cells, it is known that dermal papilla cells, which can be acquired from dermal papillae, possess hair follicle inductivity, and it has been shown that these can be acquired by *in vitro* culture thereof (Non-patent Reference No. 1).

With regard to epithelial stem cells, techniques for culturing Lgr5⁺ stem cells from intestinal epithelial tissue have been established, and regeneration of intestinal villi from Lgr5⁺ stem cells following culture has been shown to be possible (Non-patent Reference No. 2).

With respect to hair follicles, presence of stem cells possessing hair follicle inductivity in the bulge region has been suggested (Non-patent Reference No. 3); and with regard to *in vitro* growth, while there are reports of CD34⁺ integrin α6⁺ stem cell growth methods involving three-dimensional culture of murine hair follicle-derived cells (Non-patent Reference No. 4), the organ regenerativity thereof has not been sufficiently demonstrated.

Patent Reference No. 1 discloses an *in vitro* method for the production of hair follicles which comprises co-culturing dermal papilla cells with keratinocytes, with the optional addition of melanocytes, in a medium that supports hair growth, *e.g.,* Epilife, which medium may be supplemented with substances as listed therein to promote proto-hair development.

Patent Reference No. 2 discloses a method for the proliferation of hair follicle stem cells, wherein hair follicle stem cells are cultured in a culture medium for epithelial cell culture comprising an ALK5 inhibitor and a ROCK inhibitor or in a medium comprising an ALK5 inhibitor and a TGFβ inhibitor.

### PRIOR ART REFERENCES

### NON-PATENT REFERENCES

[Non-patent Reference No. 1] Tissue Eng. 13, 975-82, 2007
[Non-patent Reference No. 2] Nature. 459, 262-5, 2009
[Non-patent Reference No. 3] Exp. Cell. Res. 316, 1422-8, 2010
[Non-patent Reference No. 4] EMBO J. 36, 151-164, 2017

### PATENT REFERENCES

[Patent Reference No. 1] US 2009/198336 A1
[Patent Reference No. 2] JP 2012-249556 A

### SUMMARY OF INVENTION

### PROBLEM TO BE SOLVED BY INVENTION

At the present time, there has been no report of a technique such as would permit large-scale production of epithelial stem cells possessing hair follicle inductivity by *in vitro* culture thereof.

It is therefore an object of the present invention to provide a means for efficient growth of epithelial cells that can be used to manufacture regenerated hair follicle germs.

### MEANS FOR SOLVING PROBLEM

As a result of intensive and repeated efforts to solve the foregoing problems, the present inventor(s) established that use of a cell culture medium comprising a particular combination of additives for carrying out the culture of cells derived from epithelial tissue will permit use thereof for the manufacture of regenerated hair follicle germs, i.e., that this will make it possible to carry out large-scale production of populations of epithelial cells possessing hair follicle inductivity, which culminated in the present invention.

One aspect of the present invention provides:
[1] a culture medium for growth of epithelial cells capable of being used to manufacture regenerated hair follicle germs, the culture medium comprising basal medium and at least (1) through (3), below:
   (1) at least one species of bone morphogenetic protein (BMP) inhibitor;
   (2) at least one species of fibroblast growth factor; and
   (3) at least one species of sonic hedgehog (SHH) and/or SHH agonist.
   Various embodiments (characteristics) of the culture medium of the present invention are as follows, which embodiments are also reflected in appended claims 2-10:
[2] The culture medium according to [1], wherein
   the BMP inhibitor is selected from the group consisting of Noggin, Dorsomorphin, Chordin, Follistatin, and Ectodin.
[3] The culture medium according to [1] or [2], wherein
   the culture medium comprises at least two species of the fibroblast growth factor.
[4] The culture medium according to any of [1] through [3] wherein
   the fibroblast growth factor is selected from the group consisting of FGF-1, FGF-2, FGF-3, FGF-4, FGF-5, FGF-6, FGF-7, FGF-8, FGF-9, and FGF-10.
[5] The culture medium according to any of [1] through [4], wherein
   the SHH agonist is selected from among SAG and Purmorphamin.
[6] The culture medium according to any of [1] through [5], wherein
   the culture medium further comprises (4) at least one species of receptor tyrosine kinase ligand.
[7] The culture medium according to [6], wherein
   the receptor tyrosine kinase ligand is selected from the group consisting of EGF, TGFα, amphiregulin, and heparin-binding EGF-like growth factor (HB-EGF).
[8] The culture medium according to any of [1] through [7], wherein
   the culture medium further comprises (5) at least one species of TGFβ receptor/ALK5 inhibitor.
[9] The culture medium according to [8], wherein
   the TGFβ receptor/ALK5 inhibitor is selected from the group consisting of SB431542, KY03-I, IWR-1-endo, and A83-01.
[10] The culture medium according to any of [1] through [9], wherein
   the culture medium does not comprise Wnt or Wnt agonist, and does not comprise Notch or Notch agonist.
   A further aspect of the present invention provides:
[11] a method for growth of epithelial cells for use in manufacture of regenerated hair follicle germs, the method comprising:
   an operation in which cells derived from epithelial tissue are cultured in the culture medium according to any of [1] through [10].
   Various embodiments (characteristics) of the method of the present invention are as follows, which embodiments are also reflected in appended claims 12 and 13:
[12] The method according to [11], wherein
   the cells derived from epithelial tissue are cells obtained by causing collected epithelial tissue to undergo treatment for disaggregation into single cells.
[13] The method according to [11] or [12], wherein
   the epithelial tissue is hair follicle bulge region epithelial tissue.

Any desired combination of one or more of the characteristics of the present invention as identified herein above is also included within the scope of the present invention as defined by the appended claims.

### BENEFIT OF INVENTION

The present invention makes it possible to provide a culture medium for efficient growth of epithelial cells capable of being used to manufacture regenerated hair follicle germs, as well as a method for using the culture medium to efficiently grow such epithelial cells.

### BRIEF DESCRIPTION OF DRAWINGS

FIG. 1 shows images of cultures after 6 days of culture when murine hair follicular epithelial cells were respectively cultured in NFFSE culture medium, NFFS culture medium, and NFFS + Wnt + Notch culture medium.
FIG. 2 shows results of measurement of (A) plating efficiency, (B) fold expansion, and (C) spheroid size after 5 days of culture when murine hair follicular epithelial cells were respectively cultured in NFFSE culture medium, NFFS culture medium, and NFFS + Wnt + Notch culture medium.
FIG. 3 shows results of marker analysis when cultured murine hair follicular epithelial cells were respectively cultured in NFFSE culture medium, NFFS culture medium, and NFFS + Wnt + Notch culture medium.
FIG. 4 shows (C) quantitative results and (B) representative new hair growth when organ inductivity was evaluated through employment of (A) intracutaneous transplantation of regenerated hair follicle germs reconstructed from murine hair follicular epithelial cells respectively cultured in NFFSE culture medium, NFFS culture medium, and NFFS + Wnt + Notch culture medium. In the drawing, GT indicates guide thread, E indicates epithelial cell aggregate, and DP indicates dermal papilla cell aggregate.
FIG. 5 shows images of cultures and numbers of cells acquired per follicle after 10 days and after 20 days of culture when cultured human hair follicular epithelial cells were cultured in NFFS culture medium.
FIG. 6 shows results of stem cell marker analysis when cultured murine hair follicular epithelial cells (after 6 days of culture) and cultured human hair follicular epithelial cells (after 20 days of culture) were cultured in NFFSE culture medium or NFFS culture medium.
FIG. 7 shows (B) results of histochemical analysis and (A) experimental method for evaluation of organ inductivity of cultured human hair follicular epithelial cells cultured in NFFS culture medium.

### EMBODIMENTS FOR CARRYING OUT INVENTION

Embodiments of the present invention are described in detail below. As noted herein above, one aspect of the present invention relates to a culture medium (hereinafter also referred to as the culture medium of the present invention) for growth of epithelial cells capable of being used to manufacture regenerated hair follicle germs.

Hair follicle germs are tissue derived from hair follicles, and are made up of epithelial cells and mesenchymal cells. Hair follicle germs are formed during the embryonic stage when a portion of the epidermis thickens and the mesenchymal cells which they face agglomerate.

**In** the context of the present invention, the term "regenerated hair follicle germ" refers to a hair follicle germ that has been artificially induced and/or regenerated from epithelial cell(s) and/or mesenchymal cell(s).

**In** the context of the present invention, the term "capable of being used to manufacture regenerated hair follicle germs" means at least capable of inducing regenerated hair follicle germ(s) possessing trichogenicity when used with appropriate mesenchymal cell(s) to manufacture regenerated hair follicle germ(s). That is, in the context of the present invention, mere growth of cells derived from epithelial tissue (e.g., hair follicles), or growth of epithelial cells which induce only regenerated hair follicle germs that do not possess trichogenicity, is not intended thereby.

In the context of another embodiment of the present invention, the term "capable of being used to manufacture regenerated hair follicle germs" means permitting manufacture of regenerated hair follicle germ(s) having a high survival rate *in vivo* and possessing trichogenicity when used to manufacture regenerated hair follicle germ(s). Here, "having a high survival rate *in vivo"* may mean at least 30%, preferably at least 40%, more preferably at least 50%, more preferably at least 60%, more preferably at least 70%, more preferably at least 80%, more preferably at least 90%, or more preferably at least 95%.

A culture medium in accordance with the present invention is characterized in that it comprises basal medium and at least (1) through (3), below:
(1) at least one species of bone morphogenetic protein (BMP) inhibitor;
(2) at least one species of fibroblast growth factor; and
(3) at least one species of sonic hedgehog (SHH) and/or SHH agonist.

Basal medium refers to a culture medium containing a source of carbon, a source of nitrogen, inorganic salt(s), and so forth, which are essential for culture of cells, particularly mammalian (e.g., human) cells. As basal medium which may be used in culture media in accordance with the present invention, it is possible to use any culture medium such as may ordinarily be used in the culture of animal cells; and while there is no limitation with respect hereto, Eagle's Medium and other such minimum essential media (MEM), Dulbecco's Modified Eagle's Medium (DMEM), Minimal Essential Medium α (MEM-α), Ham's F-12 and F-10 Media, DMEM/F12 Medium, Williams' Medium E, RPMI-1640 Medium, MCDB Medium, 199 Medium, Fischer's Medium, Iscove's Modified Dulbecco's Medium (IMDM), McCoy's Modified Medium, DEF-CS Medium, CnT-PR Medium, Advanced DMEM medium, Advanced MEM medium, Advanced DMEM/F12 Medium, as well as media which are combinations hereof, may be cited as examples.

As (1) BMP inhibitor which may be used in culture media in accordance with the present invention, so long as it is capable of thwarting or inhibiting bonding of BMP molecules to BMP receptors, there is no particular limitation with respect thereto. Whether or not some molecule or compound possesses BMP inhibitory activity may be determined by using a method known to those of skill in the art (Zilberberg et al., BMC Cell Biol, 8:41, 2007) to measure BMP transcriptional activity.

As BMP inhibitor which may be used in culture media in accordance with the present invention, while there is no limitation with respect hereto, Noggin, Dorsomorphin, Chordin, Follistatin, Ectodin, and so forth, may be cited as examples. In culture media in accordance with the present invention, any one species of BMP inhibitor may be used alone or a plurality thereof may be used in combination.

Concentration of BMP inhibitor contained in culture media in accordance with the present invention may be within the range 0.1 ng/mL to 1000 ng/mL, preferably 0.3 ng/mL to 300 ng/mL, and more preferably 1 ng/mL to 100 ng/mL.

As (2) fibroblast growth factor which may be used in culture media in accordance with the present invention, while there is no limitation with respect hereto, FGF-1, FGF-2, FGF-3, FGF-4, FGF-5, FGF-6, FGF-7, FGF-8, FGF-9, and FGF-10 may be cited as examples. In culture media in accordance with the present invention, any one species of fibroblast growth factor may be used alone or a plurality thereof may be used in combination. In accordance with another embodiment of the present invention, as fibroblast growth factor, at least two species of fibroblast growth factor are used in combination.

In accordance with one embodiment of the present invention, as fibroblast growth factor, at least FGF-7 is used.

Concentration of fibroblast growth factor contained in culture media in accordance with the present invention may be within the range 0.1 ng/mL to 1000 ng/mL, preferably 0.3 ng/mL to 300 ng/mL, and more preferably 1 ng/mL to 100 ng/mL.

As (3) SHH agonist which may be used in culture media in accordance with the present invention, so long as it is capable of upregulating signaling mediated by SHH, there is no particular limitation with respect thereto. Whether or not some molecule or compound possesses SHH agonist activity may be determined, for example, by using a method such as is suggested in Japanese Patent Application Publication Kokai No. 2009-213442.

As SHH agonist which may be used in culture media in accordance with the present invention, while there is no limitation with respect hereto, SAG, and Purmorphamin may be cited as examples.

Concentration of SHH and/or SHH agonist contained in culture media in accordance with the present invention may be within the range 0.1 ng/mL to 1000 ng/mL, preferably 0.3 ng/mL to 300 ng/mL, and more preferably 1 ng/mL to 100 ng/mL.

In one embodiment, a culture medium in accordance with the present invention further comprises (4) at least one species of receptor tyrosine kinase ligand. It is preferred that a culture medium in accordance with the present invention further comprise receptor tyrosine kinase ligand due to the fact that it will permit marked improvement in survival rate when the regenerated hair follicle germ(s) that are ultimately manufactured are transplanted *in vivo.*

As receptor tyrosine kinase ligand which may be used in culture media in accordance with the present invention, while there is no limitation with respect hereto, EGF, TGFα, amphiregulin, and heparin-binding EGF-like growth factor (HB-EGF) may be cited as examples.

Concentration of receptor tyrosine kinase ligand contained in culture media in accordance with the present invention may be within the range 0.1 ng/mL to 1000 ng/mL, preferably 0.3 ng/mL to 300 ng/mL, and more preferably 1 ng/mL to 100 ng/mL.

In one embodiment, a culture medium in accordance with the present invention further comprises (5) at least one species of TGFβ receptor/ALK5 inhibitor. As TGFβ receptor/ALK5 inhibitor which may be used in culture media in accordance with the present invention, so long as it is capable of inhibiting interaction between TGFβ receptor and ALK5, there is no particular limitation with respect thereto, it being possible for this to include TGFβ receptor inhibitor, and ALK5 inhibitor.

As TGFβ receptor/ALK5 inhibitor which may be used in culture media in accordance with the present invention, while there is no limitation with respect hereto, SB431542, A83-01, ALK5 Inhibitor, D4476, LY364947, SB525334, and SD208 may be cited as examples.

Concentration of TGFβ receptor/ALK5 inhibitor contained in culture media in accordance with the present invention may be within the range 0.001 ng/mL to 1000 ng/mL, preferably 0.01 ng/mL to 100 ng/mL, and more preferably 0.1 ng/mL to 10 ng/mL.

Culture media in accordance with the present invention may, in addition to the foregoing (1) through (3), and optionally (4) and/or (5), contain other component(s) for optimization of cell culture media, and in particular of cell culture media for epithelial cells. As such other components for optimization of cell culture media, while there is no limitation with respect hereto, Glutamax^{™}, B27 supplement (GIBCO), N2 supplement (GIBCO), and other such serum substitutes, Y-27632 and other such Rock inhibitors may be cited as examples.

It is preferred that culture media in accordance with the present invention not contain additives that promote induction of differentiation and/or growth other than the foregoing (1) through (5). As such other additives, while there is no limitation with respect hereto, Wnt and Wnt agonists, and Notch and Notch agonists, may be cited as examples. Whether or not some molecule or compound possesses Wnt activity may, for example, be determined by using the method described in Korinek et al., Science 275 1784-1787, 1997 to measure Wnt transcriptional activity, and whether or not some molecule or compound possesses Notch activity may, for example, be determined by using the method described in Hsieh et al., Mol. Cell. Biol. 16, 952-959, 1996 to measure Notch transcriptional activity.

In one exemplary embodiment of the present invention, a culture medium in accordance with the present invention is substantially made up of basal medium, combination of the following additive(s), and other component(s) for optimization of cell culture media, and does not contain other additives that promote induction of differentiation and/or growth: Noggin, EGF, FGF-7, FGF-10, SAG.

In another exemplary embodiment of the present invention, a culture medium in accordance with the present invention is substantially made up of basal medium, combination of the following additive(s), and other component(s) for optimization of cell culture media, and does not contain other additives that promote induction of differentiation and/or growth: Noggin, FGF-7, FGF-10, SAG.

In the context of the present invention, note that to be "substantially made up of" some constituent(s) means that other additive(s) may be present therein to the extent that such other additive(s) do not prevent attainment of results similar to those that would be obtained were the entirety "made up of only" those constituent(s) as regards ability to manufacture regenerated hair follicle germs possessing hair follicle inductivity and/or cause growth of epithelial cells.

As noted hereinabove, another aspect of the present invention relates to a method (hereinafter also referred to as the method of the present invention) for growth of epithelial cells for use in manufacture of regenerated hair follicle germs.

A method in accordance with the present invention is characterized in that it comprises an operation in which cell(s) derived from epithelial tissue are cultured in a culture medium in accordance with the present invention.

In accordance with the present invention, cells derived from epithelial tissue might typically be prepared from hair follicles, it being possible for example to use hair follicle epithelium induced from ES cells or iPS cells, and the hair matrix, the bulge region (e.g., cells in the outermost layer of the outer root sheath in the bulge region).

As epithelial tissue which may be used in methods in accordance with the present invention, this may be collected from any of various types of animals, including not only mammalian primates (e.g., humans, monkeys, etc.), ungulates (e.g., pigs, cows, horses, etc.), and small mammalian rodents (e.g., mice, rats, rabbits, etc.), but also, e.g., dogs, and cats. With respect to collection of epithelial tissue, conditions ordinarily employed for collection of tissue may be adopted without alteration, it being possible for extraction to be carried out under sterile conditions, and for storage to be carried out using an appropriate preservative solution.

Preparation of cells derived from epithelial tissue which is from hair follicles might, for example, be carried out by causing the hair follicles which have first been isolated from the surrounding tissue to be separated into epithelial tissue and mesenchymal tissue depending on the morphology thereof. At such time, enzyme(s) may be employed for facilitating separation. As enzyme(s), dispase, collagenase, trypsin, and/or other such known substance(s) may be cited, it being possible for one of skill in the art to employ preferred enzyme(s) as appropriate.

Furthermore, as cells derived from epithelial tissue, it is possible to employ cells derived from source(s) other than hair follicles. As cells derived from source(s) other than hair follicles, while there is no limitation with respect hereto, epithelial cells from the skin or intraoral mucosa or gingiva, preferably immature epithelial precursor cells capable of differentiating into differentiated, e.g., keratinized or parakeratinized, epithelial cells from the skin or mucosa, such as, for example, nonkeratinized epithelial cells or stem cells thereof, may be cited. More specifically, examples of use of intraoral epithelial cells or primary culture cells thereof as epithelial cells are described at Japanese Patent Application Publication Kokai No. 2008-29756.

There being no particular limitation with respect to the source(s) of the ES cells which may be employed in accordance with the present invention, ES cells derived from inner cell mass(es) of all manner of animal(s) may be employed. For example, as source(s) of ES cells, ES cells derived from inner cell mass(es) of humans, mice, rats, dogs, cats, rabbits, cows, horses, sheep, goats, pigs, and/or monkeys may be employed.

"iPS cell (induced Pluripotent Stem cell)" generally refers to a cell that has been imparted with pluripotency such as will permit it to differentiate into a great many cells after the fashion of an ES cell, and with self-replicability such as will permit pluripotency to be maintained despite division and proliferation as a result of introduction, for example, of multiple types of genes and/or drugs into a somatic cell. Note, however, that the present invention is not limited to the foregoing explanation but broadly includes cells that would be considered to be "iPS cells" by those of skill in the art.

There being no particular limitation with respect to the source(s) of the iPS cells which may be employed in accordance with the present invention, iPS cells derived from all manner of animal(s) may be employed. For example, as source(s) of iPS cells, iPS cells derived from humans, mice, rats, dogs, cats, rabbits, cows, horses, sheep, goats, pigs, and/or monkeys may be employed. Furthermore, there being no particular limitation with respect to the somatic cells which serve as the source(s) of the iPS cells which may be employed in accordance with the present invention, iPS cells induced from cells derived from all manner of tissue(s) may be employed. Moreover, there being no particular limitation with respect to the method by which the iPS cells employed in accordance with the present invention may be induced, so long as it is a method permitting iPS cell(s) to be induced from somatic cell(s), iPS cells induced through employment of any sort of method may be employed.

Where cells derived from epithelial tissue collected *in vivo* are used as cells derived from epithelial tissue, it is preferred that treatment for disaggregation into single cells be carried out before these are made available for culture. In the context of the present invention, "disaggregation into single cells" refers to separation of a plurality of (typically tissue or organ) cells that are in a mutually bound or adhered state into individual cells. As such treatment for disaggregation into single cells, methods known to those of skill in the art may be employed; while there is no limitation hereto, this may be carried out by means of enzymatic treatment. The enzymes which may be used for said enzymatic treatment, and the conditions under which these may be used, are known to those of skill in the art, it being possible, for example, to use dispase, collagenase, trypsin, and/or other such enzyme(s). Disaggregation into single cells is preferred from the standpoint of efficiently promoting growth of the desired epithelial cells.

In accordance with a preferred embodiment of the present invention, the cells derived from epithelial tissue are cells derived from bulge region epithelium. Such cells might be prepared by collecting tissue from the bulge region epithelium (e.g., the outermost layer of the outer root sheath) and subjecting this to treatment for disaggregation into single cells.

Conditions ordinarily employed for culture of animal cells may be employed for culture of cells derived from epithelial tissue in culture media in accordance with the present invention, it being possible, for example, to employ culture which is carried out in an incubator having a 5% concentration of CO₂ and a temperature of approximately 37° C. Furthermore, streptomycin and/or other such antibiotic(s) may be added as appropriate.

For culture, extracellular matrices known to those of skill in the art may be employed as appropriate, it being possible, for example, to use Matrigel^{™} (Corning), Type IV Collagen (Invitrogen), Atelocollagen (Koken), Type I Collagen, and Type III Collagen.

Populations of epithelial cells grown in accordance with the foregoing methods are populations of epithelial cells possessing hair follicle inductivity, it being possible for these to be used as is to manufacture regenerated hair follicle germs, and it also being possible, where necessary, to employ techniques involving negative and/or positive selection based on surface marker(s) to further isolate particular epithelial cell populations. Techniques involving negative and/or positive selection based on surface marker(s) are also known to those of skill in the art, it being possible, for example, to employ technique(s) based on any desired antibody or antibodies, including fluorescence activated cell sorting (FACS) and/or magnetic beads separation.

Except where definitions are specifically set forth, the terminology employed in the present specification being employed for the purpose of describing specific embodiments, it is not intended that the invention should be limited thereby.

Furthermore, except where clear from context that a different sense is intended, the term "comprise" as used in present specification should be understood to mean that the item referred to (member, step, element, number, etc.) is present and not that presence of other items (members, steps, elements, numbers, etc.) is excluded.

Except where otherwise defined, all terms used herein (including technical terms and scientific terms) have the same meanings as they would be broadly understood to have by one of skill in the art to which the present invention pertains. Except where a different definition is explicitly set forth, the terminology used herein should be interpreted as having a meaning that is consistent with the meaning employed in the present specification and related field(s) of art, and should not be construed in an idealized or overly formal sense.

Below, the present invention is described in further detail with reference to working examples. Note, however, that as the present invention is capable of being embodied in a wide variety of forms, it should not be construed as being limited by the working examples that are described herein.

### WORKING EXAMPLES

### (Working Example 1) Method for Culturing Murine Hair Follicular Epithelial Cells

### 1) Experimental Animals

Hair follicles were collected from C57BL/6 mice (CLEA Japan) and C57BL/6 6-TgN (act-EGFP) mice of age 7-8 weeks. Furthermore, skin containing body hair germs was collected from C57BL/6 6-TgN (act-EGFP) mice (SLC) of embryonic age 18.0 to 18.5 days. Furthermore, regenerated hair follicle germs prepared in accordance with the following experimental procedure were grafted onto Balb/c nu/nu mice (SLC) of age 6 to 8 weeks.

### 2) Preparation of Hair Follicular Epithelial Cells

A 25G injection needle was used to remove the collagen sheath from whisker tissue extracted from C57BL/6 EGFP mice, and this was subdivided into a portion corresponding to the bulge region. The bulge region tissue was allowed to react for 4 minutes at 37 °C in a solution in which final concentration of Dispase II (Becton Dickinson) was 4.8 U/ml and of Collagenase (Worthington; Lakewood NJ) was 100 U/ml, following which a 25G injection needle was used to surgically separate this into epithelial tissue from the bulge region and mesenchymal tissue from an area peripheral to the bulge. The separated bulge region epithelial tissue was subjected to enzymatic treatment for 1 hour in an incubator with 0.05% Trypsin (Invitrogen; Carlsbad US), disaggregation into single cells being carried out by causing this to pass through a cell strainer having 35-µm pores.

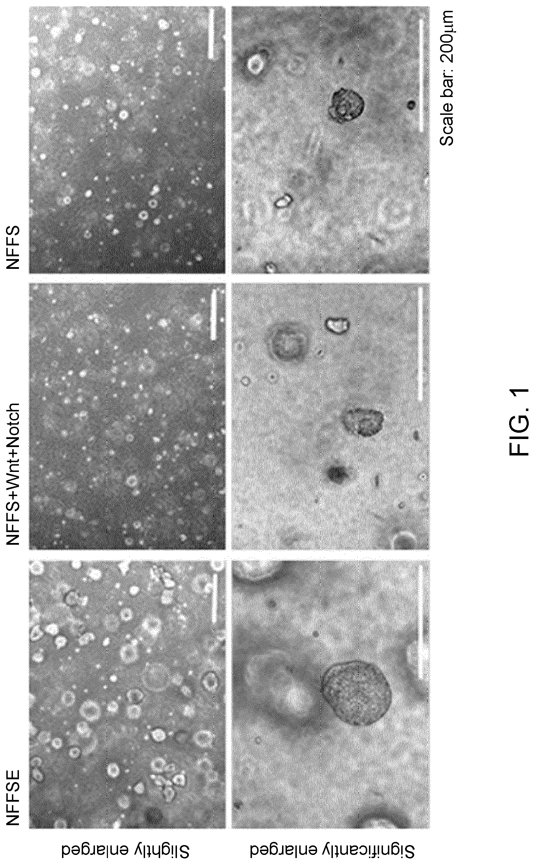

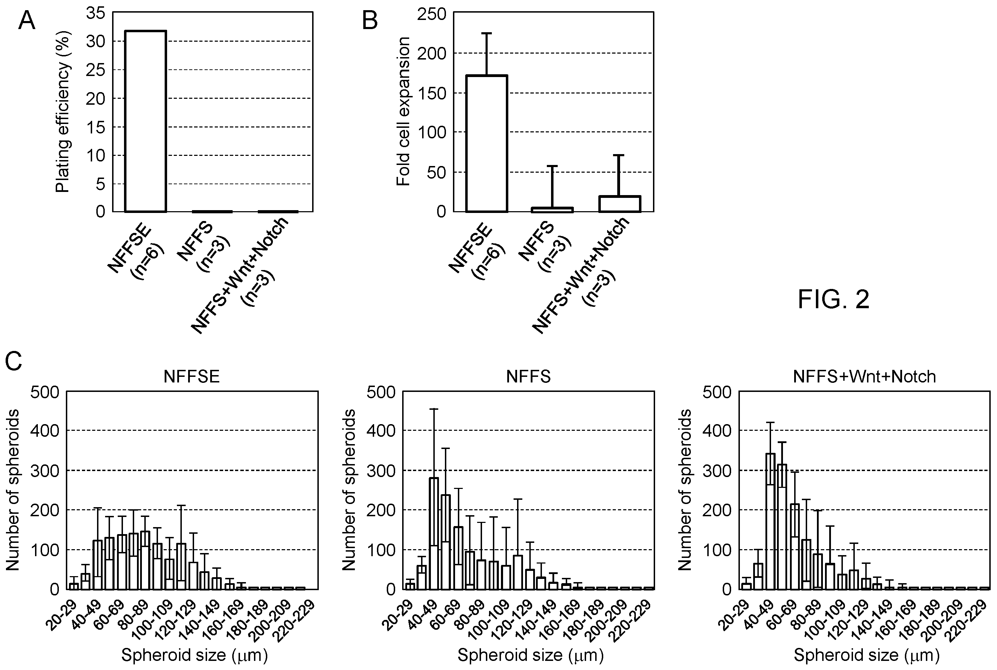

Culture of the disaggregated single cells was carried out using ADVANCED DMEM/F-12 (ThermoFisher) as basal medium, with Matrigel (Corning) being employed as scaffolding, by causing the cells to be suspended in a culture medium (NFFSE culture medium) containing additives in the form of B27 supplement (GIBCO), N2 supplement (GIBCO), Rock inhibitor (Y27632; WAKO), EGF (Peprotech), FGF-7 (R&D), FGF-10 (R&D), SHH agonist (SAG; cayman), and BMP inhibitor (Noggin; Peprotech), as well as 1% Penicillin-Streptomycin (GIBCO); in a culture medium (NFFS culture medium) identical to NFFSE culture medium except that EGF was not present; and in a culture medium (NFFS + Wnt + Notch culture medium) identical to NFFS culture medium except that Wnt3a (R&D) and Notch ligand (Jagged2 and DLL1) (R&D) were additionally present, following which three-dimensional culture was carried out in a 6-well plate. Culture images are shown in FIG. 1, results of measurement of plating efficiency, spheroid size, and fold expansion after 5 days of culture being shown in FIG. 2. Under conditions other than those employed for NFFSE culture medium, smaller colonies were formed.

Gel containing cultured hair follicular epithelial cells at which three-dimensional culture was carried out were recovered, Collagenase I (Worthington) digestion, Trypsin (GIBCO) digestion, and DNase Type I (Worthington) digestion were carried out, and following suspension in culture medium, this was made to pass through a 35-µm cell strainer to obtain cultured murine hair follicular epithelial cells.

### 3) Analysis of Differentiation of Cultured Hair Follicular Epithelial Cells Derived from Mouse Hair Follicles

Before evaluating hair follicular epithelial cell differentiability, real-time PCR analysis was carried out to evaluate differentiation of hair follicular epithelial cells following culture.

Extraction of RNA from cultured hair follicular epithelial cells was carried out using an RNeasy Plus Micro Kit (QUIAGEN), a SuperScript VILO MasterMix (Invitrogen) being used to synthesize cDNA from the RNA that was obtained. Real-time PCR was carried out using the foregoing cDNA as template with a TaqManFast Advanced Master Mix (applied biosystem) and liquid mixture of Taqman primers for the various markers in a QuantStudio 12K Flex (applied biosystem) to cause the PCR reaction (20 seconds at 95 °C, (1 second at 95 °C, 20 seconds at 60 °C) × 40 cycles), and the degree to which marker genes were expressed was analyzed.

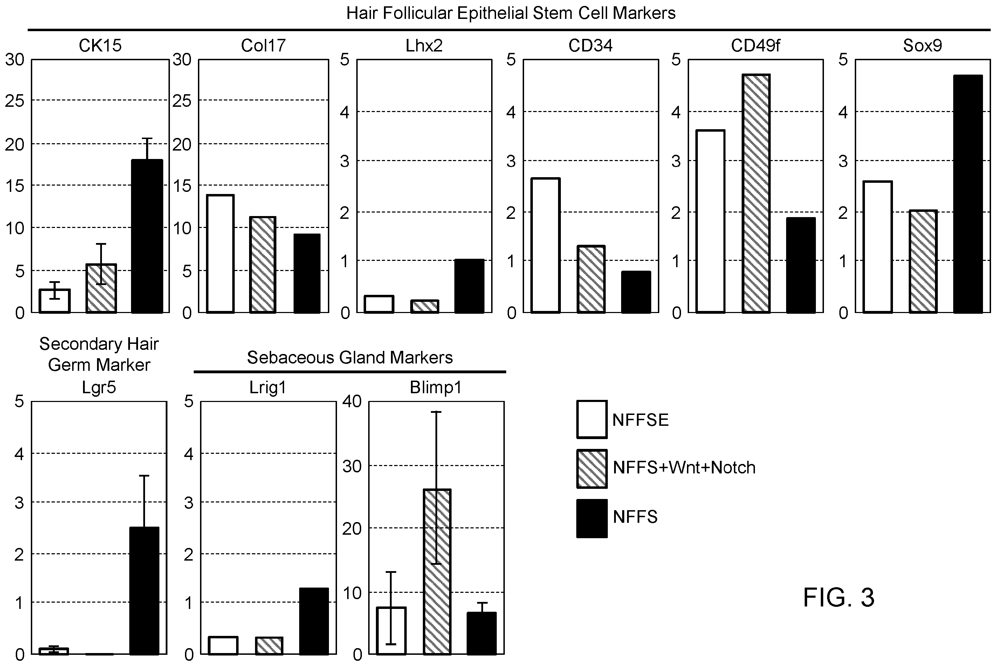

Results of analysis of expression of differentiation markers and stem cell markers for hair follicular epithelial cells cultured using NFFSE culture medium, NFFS culture medium, or NFFS + Wnt + Notch culture medium are shown in FIG. 3. For cells cultured in NFFS culture medium, secondary hair germ markers (Lgr5 and P-cadherin) and hair bulb marker (Lef1) were expressed to a high degree; for cells cultured in NFFS + Wnt + Notch culture medium, strong induction of sebaceous gland markers (Blimp1, c-myc, and PPAR-γ) and epidermis markers (CK1 and Loricrin) was observed; for cells cultured in NFFSE culture medium, levels of CD34, CD49f, and other such stem cell markers were maintained.

### 4) Evaluation of Organ Inductivity of Murine Hair Follicular Epithelial Cells

To evaluate the organ inductivity of cultured hair follicular epithelial cells, the organ bud generation method was used to reconstruct regenerated hair follicle germs, analysis being carried out using trichogenicity following transplantation in animals as an indicator.

Preparation of regenerated hair follicle germs was carried out in accordance with the organ bud generation method (Japanese Patent No. 5932671). That is, cultured dermal papilla cells and the murine hair follicular epithelial cells that were obtained were respectively and individually transferred to 1.5-ml microtubes (Eppendorf) coated with silicone grease (Dow Corning Toray), whereupon centrifugal separation was carried out, with the precipitated fraction being recovered, and a 0.5-20-µl GELoader Tip (Eppendorf) being used to completely remove the supernatant culture solution following centrifugation. Next, 30 ml of Cell matrix type I-A (Nitta gelatin; Osaka, Japan) was dripped into a petri dish coated with silicone grease to prepare a collagen gel drop, and a 0.1-10-µl pipette tip (Quality Scientific plastics) was used to inoculate this with on the order of 0.2 µl of the cultured dermal papilla cells that were prepared above to prepare a cell aggregate. A 0.1-10-µl pipette tip (Quality Scientific plastics) was then used to inoculate the interior of same gel drop with on the order of 0.2 µl of the epithelial cells that were prepared above, in such fashion as to cause this to adhere to the cultured dermal papilla cell aggregate, to prepare a cell aggregate. Moreover, while using a stereoscopic microscope to confirm proper placement, a nylon thread (Matsuda Ika Kogyo) of total length 5 mm was inserted from the epithelial cell side of the cell aggregate comprising cultured dermal papilla cells and epithelial cells in such fashion as to vertically penetrate the contact surface between the cultured dermal papilla cell fraction and the epithelial cell fraction without disturbing the structure (particularly the contact surface between the epithelial cells and the mesenchymal cells) of the cell aggregate, following which this was allowed to stand undisturbed for 5 minutes at 37 °C and the gel drop was allowed to solidify so as to cause the bond between the epithelial cells and the mesenchymal cells to be further strengthened to prepare a regenerated hair follicle germ having a guide.

Following culture for 8 to 16 hours, intracutaneous transplantation of the hair follicle germ into the skin of a mouse was carried out in accordance with the conventional method. That is, a nude mouse was anesthetized in the usual way, and following disinfection of the dorsum with Isodine, was made to assume a naturally recumbent posture. A V-lance microscalpel (Alcon Japan) was used to pierce the skin, the graft which was formed extending from the epidermal layer of the skin to the subdermal layer. The graft extended to a depth of approximately 400 µm in a vertical direction from the body surface, being on the order of approximately 1 mm in the horizontal direction. Sharp Tweezers No. 5 (Natsume Seisakusho) were used to cause the regenerated hair follicle germ in which the guide made of nylon thread was inserted to be inserted therein in such fashion as to cause the epithelial cell component to be directed toward the body surface side of the graft. Transplanted depth was adjusted so as to cause the top portion of the epithelial cell component of the regenerated hair follicle germ to be exposed at the top portion of the graft, the guide made of nylon thread being located so as to be exposed at the body surface. The guide made of nylon thread was secured by SteriStrip (3M) to the surface of the skin near the graft, following which Nurseban and surgical tape (3M) were used to protect the graft. 5 to 7 days following transplant, the protective tape was removed, and the guide made of nylon thread was allowed to remain at the transplanted location, but was removed therefrom 1 day thereafter if it still remained. Survival of the transplanted matter was determined by visual inspection or fluorescence stereomicroscopy, after which follow-up observation was carried out. Follow-up observation was carried out by using visual inspection, fluorescence stereomicroscopy, and stereoscopic microscopy to inspect and photograph the transplanted location on the anesthetized mouse to evaluate new hair growth of the regenerated hair follicle.

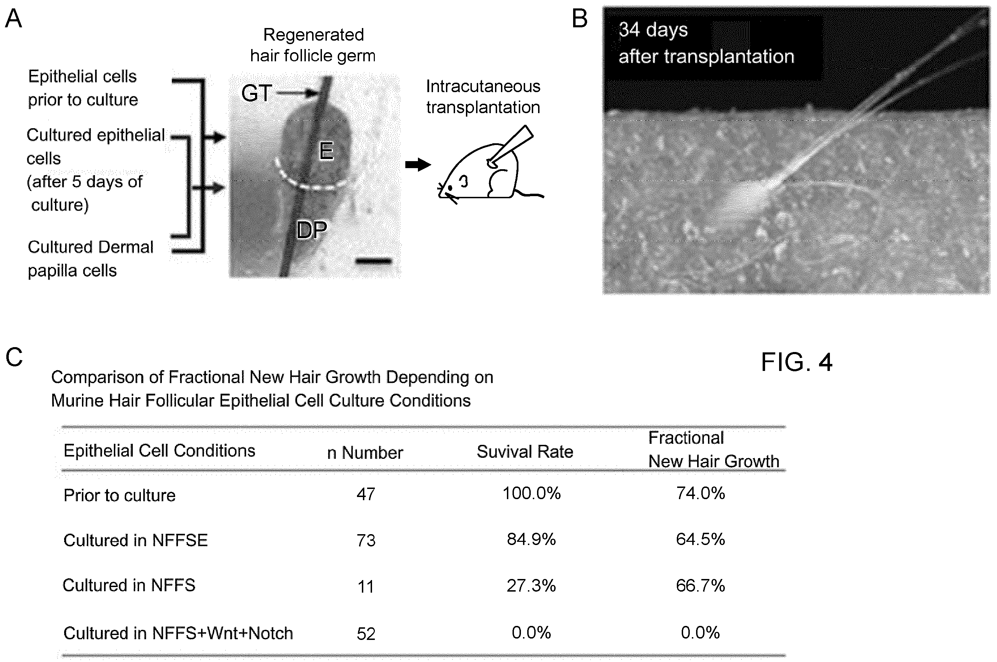

As a result, while new hair growth was observed at cells cultured in NFFSE culture medium and in NFFS culture medium, new hair growth was not observed at cells cultured in NFFS + Wnt + Notch culture medium, it being indicated that culture of hair follicular epithelial cells possessing organ inductivity was possible with NFFSE or NFFS culture medium (FIG. 4).

### (Working Example 2) Culture of Hair Follicular Epithelial Cells Derived from Human Hair Follicles

### 1) Recovery of Hair Follicle Bulge Epithelial Cells from Human Scalp Tissue

Extracted human scalp tissue was sequentially washed using povidone iodine (Meiji Seika Pharma), PBS (Nacalai Tesque), and tissue recovery solution (DMEM containing 1% HEPES, 50 µg/mL gentamicin, and 10% bovine serum), in that order. A scalpel was used to cut this into strips, each of which comprised hair follicles lined up in a single row, following which each strip was further separated into top and bottom along the surface at the boundary between the dermal layer and the subcutaneous fat layer. The dermal layer (upper tissue) which included bulge epithelial cells was recovered into a culture dish, and the upper tissue was cut into fine pieces, each of which was made to comprise a single follicle pore. 1000 U/mL Dispase (Wako Pure Chemical) and Collagenase (Worthington) were mixed and added to the tissue that had been disaggregated into single hair follicles, and this was incubated for 10 to 20 minutes at 37 °C. After washing with 2 mL of tissue recovery solution, 2 mL of tissue recovery solution and 1 µL of DNase I (Worthington) were added thereto, and this was allowed to stand undisturbed for 1 to 2 minutes at room temperature. Tweezers were used to pull out the hair follicle in upward fashion, and the lower portion produced as a result of cutting below the sebaceous gland was collected in a centrifuge tube. The lower portion was washed twice with PBS by centrifugation. 100 µL of 2.5% Trypsin (SAFC Bioscience) and 4.9 mL of PBS were mixed and added thereto, and this was incubated for 15 minutes at 37 °C, following which 5 mL of tissue recovery solution and 5 µL of DNase I were added and gently mixed therewith. The cell suspension that was obtained was made to pass through a 40-µm-diameter cell strainer, addition of 2 mL of tissue recovery solution from a location above the strainer being carried out twice. The cell suspension was centrifuged for 3 minutes at 4 °C and 590 g, the supernatant was discarded, and 1 mL of tissue recovery solution was added thereto. Cell concentration was measured, following which an amount of the cell suspension sufficient to obtain 6 × 10⁴ cells/tube was transferred to a 1.5-mL Eppendorf tube. The tube was centrifuged for 3 minutes at 4 °C and 310 g, the supernatant was discarded, and 600 µL/tube of a 1% solution of Atelocollagen (Koken) was added thereto and mixed therewith. Following centrifugation to remove bubbles, the wells of a 6-well plate were respectively seeded with 90 µL/well of the cell suspension. After allowing the gel to solidify for on the order of 30 minutes within an incubator, 3 mL/well of human NFFSE culture medium having the following composition or NFFS culture medium which was identical to NFFSE culture medium except that EGF was not present was added thereto, and culture was initiated in a CO₂ incubator: ADVANCED DMEM/F-12 (ThermoFisher), 1 M HEPES, Glutamax (Thermo Fisher Scientific), N2 supplement (Thermo Fisher Scientific), B27 supplement (Thermo Fisher Scientific), 10 mM Y-27632 (Wako Pure Chemical), 250 µg/mL Noggin (Sigma), 100 µg/mL FGF-7 (Millipore), 100 µg/mL FGF-10 (Wako Pure Chemical), 20 ng/mL EGF (Peprotech), and 500 µg/mL SAG (Millipore).

### 2) Human Hair Follicle Bulge Epithelial Cell Subcultures

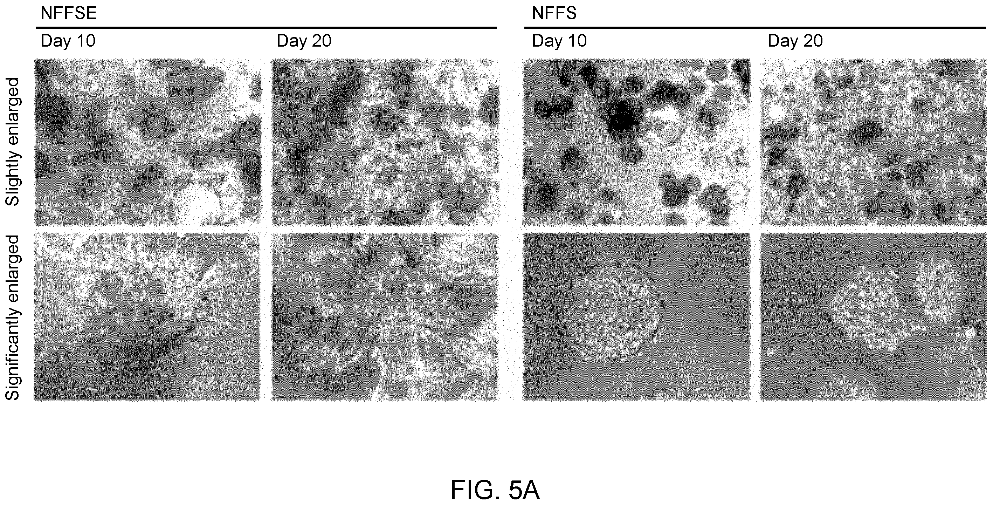

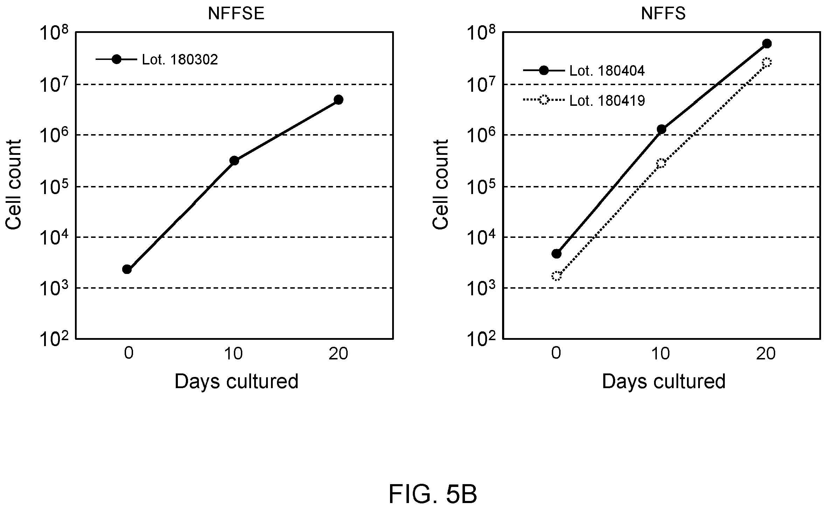

A cell scraper was used to cause the gel at which cells had formed colonies to be scraped out of the culture dish. The gel that was scraped therefrom was recovered in a 50-mL tube, and 10 mL of human epithelial cell culture medium (see above) was added thereto. The gel was physically macerated by pipetting, and collagenase was added thereto. This was incubated for a maximum time of 60 minutes at 37 °C until the gel was completely dissolved. 10 mL of PBS was added thereto, this was centrifuged for 3 minutes at 4° C and 590 g, and the supernatant was discarded, the foregoing being carried out three times. After loosening the pellet by tapping, a solution of 0.125% trypsin/10 µg/mL DNase I was added thereto, and this was incubated for 15 minutes at 37 °C. 10 mL of human epithelial cell culture medium was added thereto, this was centrifuged for 3 minutes at 4 °C and 590 g, and the supernatant was discarded. After loosening the cells by tapping, human epithelial cell culture medium containing 5 µL of DNase I was added thereto. The cell suspension that was obtained was made to pass through a 40-µm-diameter cell strainer, and this was centrifuged for 3 minutes at 4 °C and 590 g. After discarding the supernatant, 1 mL of human epithelial cell culture medium was added thereto to obtain a cell suspension. Cell concentration was measured, following which an amount of the cell suspension sufficient to obtain 6 × 10⁴ cells/tube was transferred to a 1.5-mL Eppendorf tube. The tube was centrifuged for 3 minutes at 4 °C and 310 g, the supernatant was discarded, and 600 µL/tube of a 1% solution of Atelocollagen was added thereto and mixed therewith. Following centrifugation to remove bubbles, the wells of a 6-well plate were respectively seeded with 90 µL/well of the cell suspension. After allowing the gel to solidify for on the order of 30 minutes within an incubator, 3 mL/well of human epithelial cell culture medium was added thereto, and this was further cultured in a CO₂ incubator. Numbers of cells acquired per follicle and culture images after 10 days and 20 days of culture are shown at FIG. 5.

### 3) Analysis of Differentiation of Cultured Hair Follicular Epithelial Cells Derived from Human Hair Follicles

Differentiation of cultured hair follicular epithelial cells derived from humans was evaluated by flow cytometric analysis.

### 3-1) Intracellular Marker Analysis

The cultured hair follicular epithelial cells (after 20 days of culture) were suspended in PBS - 0.2 mM EDTA - 0.05% BSA, following which this was centrifuged at 4 °C for 5 minutes at 590 g, the supernatant was discarded, and the cells were resuspended in 4% PFA or 80% MeOH. The cell suspension was incubated for 10 minutes at 4 °C to immobilize intracellular protein. Following immobilization, the cells were resuspended in PBS - 0.2 mM EDTA - 0.05% BSA, following which washing at 4 °C for 5 minutes at 1100 g) was carried out twice, following which this was resuspended in 0.1% Triton X-100, and this was incubated for 10 minutes at 4 °C to cause permeabilization thereof. Following permeabilization, the cells were washed twice, following which they were resuspended in a PBS - 0.2 mM EDTA - 0.05% BSA solution containing PE-Cy7-labeled antibody specific for human CD34 (BD) or eFlour 660-labeled antibody specific for human CD49f (eBioScience), and this was incubated for 15 minutes at 4 °C. After being allowed to react, the cells were washed twice, following which a flow cytometer was used to carry out analysis thereof. A procedure similar to that described above was also performed on cells derived from cultured murine whisker tissue.

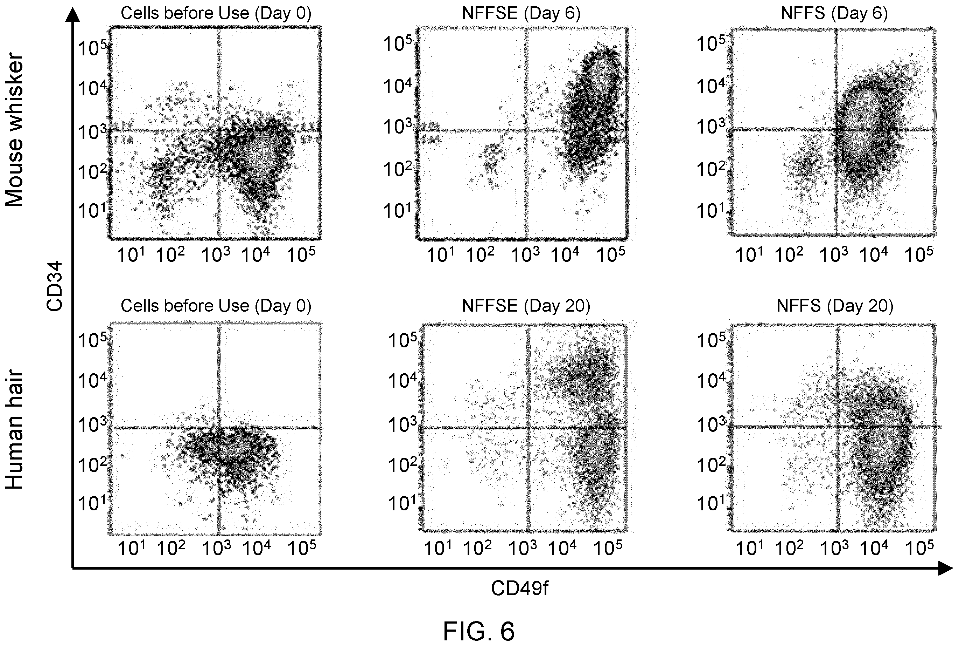

Results are shown in FIG. 6. It was found that the cultured hair follicular epithelial cells comprised cell populations including those which were CD34⁺ and CD49f⁺ (FIG. 6).

### 4) Evaluation of Organ Inductivity of Human Hair Follicular Epithelial Cells

To evaluate the organ inductivity of cultured human hair follicular epithelial cells, mouse embryonic skin mesenchymal tissue known to possess organ inductivity was used to carry out the organ bud generation method to reconstruct regenerated hair follicle germs, analysis being carried out using hair growth capability following *in vitro* culture as an indicator.

Skin mesenchymal cells from mouse embryos were prepared in accordance with the conventional method. That is, dorsal skin was collected from EGFP mouse embryos of embryonic age 18.0 to 18.5 days, and the method reported by Nakao et al. (Nakao K et al., Nat. Methods, 4(3), 227-30, 2007) was partially modified, dispase treatment being carried out while subjected to shaking under conditions of 55 rpm and 4 °C for 1 hour to cause separation of the epithelial layer and the dermal layer. The dermal layer was further twice subjected to treatment with 100 units/ml of collagenase I (Worthington) for 40 minutes at 37 °C, following which treatment for disaggregation into single cells was carried out to obtain mouse embryonic skin mesenchymal cells. The mouse embryonic skin mesenchymal cells and the human hair follicular epithelial cells that were obtained were respectively and individually transferred to 1.5-ml microtubes (Eppendorf) coated with silicone grease, whereupon centrifugal separation was carried out, with the precipitated fraction being recovered, and a 0.5-20-µl GELoader Tip (Eppendorf) being used to completely remove the supernatant culture solution following centrifugation. Next, 30 µl of Cell matrix type I-A (Nitta gelatin; Osaka, Japan) was dripped into a petri dish coated with silicone grease to prepare a collagen gel drop, and a 0.1-10-µl pipette tip (Quality Scientific plastics) was used to inoculate this with on the order of 0.2 µl of the cultured dermal papilla cells that were prepared above to prepare a cell aggregate. A 0.1-10-µl pipette tip (Quality Scientific plastics) was then used to inoculate the interior of same gel drop with on the order of 0.2 µl of the epithelial cells that were prepared above, in such fashion as to cause this to adhere to the cultured dermal papilla cell aggregate, to prepare a cell aggregate. Moreover, while using a stereoscopic microscope to confirm proper placement, a nylon thread (Matsuda Ika Kogyo) of total length 5 mm was inserted from the epithelial cell side of the cell aggregate comprising cultured dermal papilla cells and epithelial cells in such fashion as to vertically penetrate the contact surface between the cultured dermal papilla cell fraction and the epithelial cell fraction without disturbing the structure (particularly the contact surface between the epithelial cells and the mesenchymal cells) of the cell aggregate, the collagen gel was transferred in its entirety to a location over a 0.4 ml pore size Cell Culture Insert (Becton Dickinson) that had been placed on a 6 well Plate (Becton Dickinson) in which 1 ml of DMEM 10% FBS had been added, organ culture was carried out for 7 days in a 37 °C/5% CO₂ incubator to prepare regenerated hair follicle germs.

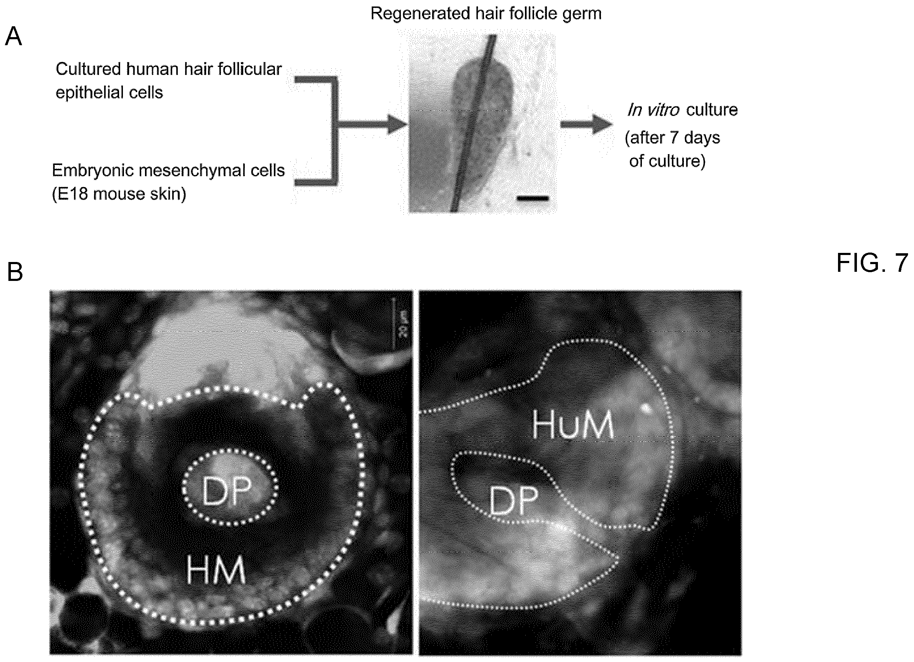

Following culture, the regenerated hair follicles were extracted from the gel and were fixed with 4% paraformaldehyde, following which a tissue processor (Leica; ASP300S) and an embedding device (Sakura; Tissue-Tek TEC) were used to carry out paraffin embedding, following which a microtome was used to prepare 5-µm section, and an antibody specific for human mitochondria (Millipore) was used to carry out immunostaining. An LSM-780 confocal laser microscope (Carl-Zeiss) was used to analyze the immunostained section. Results are shown in FIG. 7. After 7 days of culture, a hair follicle tissue-like structure was indicated in which human-derived cells were arranged in such fashion as to surround mouse-derived cells, and in which hair matrix cells surrounded dermal papilla cells. Based on the foregoing results, it is thought that cultured human hair follicular epithelial cells formed hair follicle tissue as a result of an epithelialmesenchymal interaction with cells derived from mouse embryonic skin mesenchymal tissue, it being indicated that cultured human hair follicular epithelial cells possess organ inductivity.

## Claims

1. A culture medium for growth of epithelial cells capable of being used to manufacture regenerated hair follicle germs, the culture medium comprising basal medium and at least (1) through (3), below:
(1) at least one species of bone morphogenetic protein (BMP) inhibitor;
(2) at least one species of fibroblast growth factor; and
(3) at least one species of sonic hedgehog (SHH) and/or SHH agonist.

2. The culture medium according to claim 1, wherein
the BMP inhibitor is selected from the group consisting of Noggin, Dorsomorphin, Chordin, Follistatin, and Ectodin.

3. The culture medium according to claim 1 or claim 2, wherein
the culture medium comprises at least two species of the fibroblast growth factor.

4. The culture medium according to any one of claims 1 through 3, wherein
the fibroblast growth factor is selected from the group consisting of FGF-1, FGF-2, FGF-3, FGF-4, FGF-5, FGF-6, FGF-7, FGF-8, FGF-9, and FGF-10.

5. The culture medium according to any one of claims 1 through 4, wherein
the SHH agonist is selected from among SAG and Purmorphamin.

6. The culture medium according to any one of claims 1 through 5, wherein
the culture medium further comprises (4) at least one species of receptor tyrosine kinase ligand.

7. The culture medium according to claim 6, wherein
the receptor tyrosine kinase ligand is selected from the group consisting of EGF, TGFα, amphiregulin, and heparin-binding EGF-like growth factor.

8. The culture medium according to any one of claims 1 through 7, wherein
the culture medium further comprises (5) at least one species of TGFβ receptor/ALK5 inhibitor.

9. The culture medium according to claim 8, wherein
the TGFβ receptor/ALK5 inhibitor is selected from the group consisting of SB431542, KY03-I, IWR-1-endo, and A83-01.

10. The culture medium according to any one of claims 1 through 9, wherein
the culture medium does not comprise Wnt or Wnt agonist, and does not comprise Notch or Notch agonist.

11. A method for growth of epithelial cells for use in manufacture of regenerated hair follicle germs, the method comprising:
an operation in which cells derived from epithelial tissue are cultured in the culture medium according to any one of claims 1 through 10.

12. The method according to claim 11, wherein
the cells derived from epithelial tissue are cells obtained by causing collected epithelial tissue to undergo treatment for disaggregation into single cells.

13. The method according to claim 11 or claim 12, wherein
the epithelial tissue is hair follicle bulge region epithelial tissue.

## Patentansprüche

1. Kulturmedium für das Wachstum von Epithelzellen, geeignet für die Verwendung bei der Herstellung von regenerierten Haarfollikelkeimen, wobei das Kulturmedium Basalmedium und mindestens die nachfolgenden (1) bis (3) umfasst:
(1) mindestens eine Art eines Inhibitors für das morphogenetische Knochenprotein (*bone morphogenetic protein* - BMP);
(2) mindestens eine Art von Fibroblasten-Wachstumsfaktor; und
(3) mindestens eine Art von Sonic Hedgehog (SHH) und/oder SHH-Agonist.

2. Kulturmedium nach Anspruch 1, wobei
der BMP-Inhibitor ausgewählt ist aus der Gruppe bestehend aus Noggin, Dorsomorphin, Chordin, Follistatin und Ectodin.

3. Kulturmedium nach Anspruch 1 oder Anspruch 2, wobei
das Kulturmedium mindestens zwei Arten des Fibroblasten-Wachstumsfaktors umfasst.

4. Kulturmedium nach einem der Ansprüche 1 bis 3, wobei
der Fibroblasten-Wachstumsfaktor ausgewählt ist aus der Gruppe bestehend aus FGF-1, FGF-2, FGF-3, FGF-4, FGF-5, FGF-6, FGF-7, FGF-8, FGF-9 und FGF-10.

5. Kulturmedium nach einem der Ansprüche 1 bis 4, wobei
der SHH-Agonist ausgewählt ist aus SAG und Purmorphamin.

6. Kulturmedium nach einem der Ansprüche 1 bis 5, wobei
das Kulturmedium ferner (4) mindestens eine Art von Rezeptor-Tyrosinkinase-Ligand umfasst.

7. Kulturmedium nach Anspruch 6, wobei
der Rezeptor-Tyrosinkinase-Ligand ausgewählt ist aus der Gruppe bestehend aus EGF, TGFα, Amphiregulin und dem heparinbindenden EGF-ähnlichen Wachstumsfaktor.

8. Kulturmedium nach einem der Ansprüche 1 bis 7, wobei
das Kulturmedium ferner (5) mindestens eine Art von TGFβ-Rezeptor/ALK5-Inhibitor umfasst.

9. Kulturmedium nach Anspruch 8, wobei
der TGFB-Rezeptor/ALK5-Inhibitor ausgewählt ist aus der Gruppe bestehend aus SB431542, KY03-I, IWR-1-endo und A83-01.

10. Kulturmedium nach einem der Ansprüche 1 bis 9, wobei
das Kulturmedium kein Wnt oder Wnt-Agonist und kein Notch oder Notch-Agonist umfasst.

11. Verfahren zum Wachstum von Epithelzellen zur Verwendung bei der Herstellung von regenerierten Haarfollikelkeimen, wobei das Verfahren umfasst:
einen Vorgang, bei dem aus Epithelgewebe stammende Zellen in dem Kulturmedium nach einem der Ansprüche 1 bis 10 kultiviert werden.

12. Verfahren nach Anspruch 11,
wobei die aus Epithelgewebe stammenden Zellen Zellen sind,
erhalten durch Veranlassen gesammelten Epithelgewebes sich einer Behandlung zur Disaggregation in einzelne Zellen zu unterziehen.

13. Verfahren nach Anspruch 11 oder Anspruch 12, wobei
das Epithelgewebe Epithelgewebe der Haarfollikel-Wulstregion ist.

## Revendications

1. Milieu de culture pour la croissance de cellules épithéliales pouvant être utilisées pour fabriquer des germes de follicules pileux régénérés, le milieu de culture comprenant un milieu de base et au moins (1) à (3), ci-dessous :
(1) au moins une espèce d'inhibiteur de protéine morphogénétique osseuse (BMP) ;
(2) au moins une espèce de facteur de croissance des fibroblastes ; et
(3) au moins une espèce de sonic hedgehog (SHH) et/ou d'agoniste de SHH.

2. Milieu de culture selon la revendication 1, dans lequel
l'inhibiteur de BMP est choisi dans le groupe constitué par la noggine, la dorsomorphine, la chordine, la follistatine et l'ectodine.

3. Milieu de culture selon la revendication 1 ou la revendication 2, dans lequel
le milieu de culture comprend au moins deux espèces du facteur de croissance des fibroblastes.

4. Milieu de culture selon l'une quelconque des revendications 1 à 3, dans lequel
le facteur de croissance des fibroblastes est choisi dans le groupe constitué par FGF-1, FGF2, FGF-3, FGF-4, FGF-5, FGF-6, FGF-7, FGF-8, FGF-9 et FGF-10.

5. Milieu de culture selon l'une quelconque des revendications 1 à 4, dans lequel
l'agoniste de SHH est choisi parmi SAG et Purmorphamine.

6. Milieu de culture selon l'une quelconque des revendications 1 à 5, dans lequel le milieu de culture comprend en outre (4) au moins une espèce de ligand de récepteur tyrosine kinase.

7. Milieu de culture selon la revendication 6, dans lequel le ligand de récepteur tyrosine kinase est choisi dans le groupe constitué par EGF, TGFα, amphiréguline et facteur de croissance de type EGF se liant à l'héparine.

8. Milieu de culture selon l'une quelconque des revendications 1 à 7, dans lequel le milieu de culture comprend en outre (5) au moins une espèce d'inhibiteur de récepteur de TGFβ/d'ALK5.

9. Milieu de culture selon la revendication 8, dans lequel l'inhibiteur de récepteur de TGFβ/d'ALK5 est choisi dans le groupe constitué de SB431542, KY03-I, IWR-1-endo et A83-01.

10. Milieu de culture selon l'une quelconque des revendications 1 à 9, dans lequel le milieu de culture ne comprend pas de Wnt ou d'agoniste de Wnt, et ne comprend pas de Notch ou d'agoniste de Notch.

11. Procédé de croissance de cellules épithéliales destinées à être utilisées dans la fabrication de germes de follicules pileux régénérés, le procédé comprenant :
une opération dans laquelle des cellules dérivées de tissu épithélial sont cultivées dans le milieu de culture selon l'une quelconque des revendications 1 à 10.

12. Procédé selon la revendication 11, dans lequel
les cellules dérivées de tissu épithélial sont des cellules obtenues en faisant subir au tissu épithélial collecté un traitement de désagrégation en cellules individuelles.

13. Procédé selon la revendication 11 ou la revendication 12, dans lequel
le tissu épithélial est un tissu épithélial de la région du renflement du follicule pileux.
